# EUROPÄISCHE PATENTSCHRIFT

(11) **EP 0 983 985 B1**
(45) Veröffentlichungstag und Bekanntmachung des Hinweises auf die Patenterteilung: **10.12.2003**
(21) Anmeldenummer: 99116582.0
(22) Anmeldetag: 25.08.1999
(51) Int. Cl.: C07C 29/90, C07C 29/132

(54) **Verfahren zur Reduzierung des Gehalts an Acetalen oder Ketalen in alkoholischen Reaktionsgemischen**
Method for the reduction of the acetal or ketal content in alcoholic reaction mixtures
Méthode pour la réduction de la teneur en acétals ou kétals dans des mélanges réactionnels

(30) Priorität: 04.09.1998 DE 19840277
(43) Veröffentlichungstag der Anmeldung: 08.03.2000
(73) Patentinhaber: Degussa AG, 40474 Düsseldorf (DE)
(72) Erfinder: Haas, Thomas, Dr., 60316 Frankfurt (DE); Jaeger, Bernd, Dr., 64297 Darmstadt (DE); Sauer, Jörg, Dr., 63517 Rodenbach (DE); Vanheertum, Rudolf, Dr., 63796 Kahl (DE)

(56) Entgegenhaltungen:
- EP-A- 0 572 812
- WO-A-97/01523
- WO-A-97/36846
- DE-A- 4 220 939
- US-A- 2 569 671
- US-A- 3 819 728
- US-A- 4 401 834

## Beschreibung

Die Erfindung betrifft ein Verfahren zur Reduzierung des Gehalts an Acetalen oder Ketalen in einem wäßrigen Reaktionsgemisch, das pro Mol des Acetals oder Ketals mindestens 10 Mol eines ein- oder mehrwertigen Alkohols enthält, durch heterogen-katalytische Hydrierung.

Während der Hydrierung von Aldehyden und Ketonen zu Alkoholen kommt es, wenn die Hydrierung unterhalb von pH 7 oder in Gegenwart von saure Zentren aufweisenden Feststoffkatalysatoren durchgeführt wird, leicht zur Bildung von Acetalen und Ketalen, die unter milden Hydrierbedingungen nicht befriedigend in den zugrundeliegenden und aus der Carbonylverbindung durch Hydrierung gebildeten Alkohol überführt werden können. Insbesondere bei der Hydrierung von α- und β-Hydroxycarbonylverbindungen zu 1,2- beziehungsweise 1,3-Diolen entstehen stabile cyclische Acetale beziehungsweise Ketale, nämlich 2-substituierte 1,3-Dioxolanbeziehungsweise 1,3-Dioxanderivate. Um derartige Acetale und Ketale zu hydrieren, sind höhere Temperaturen und/oder spezielle Katalysatoren erforderlich. Ein Problem bei der Reduzierung des Gehalts an Acetalen und Ketalen in wäßrig alkoholischen Reaktionsgemischen unterschiedlicher Provenienz besteht zusätzlich auch darin, daß unter den erforderlichen härteren Hydrierbedingungen die Gefahr besteht, daß der Alkohol selbst zersetzt wird.

Im Verfahren der EP-A 0 572 812 wird 3-Hydroxypropionaldehyd zu 1,3-Propandiol reduziert, wobei sich an eine erste Hydrierstufe zum Zwecke der Reduzierung des Restcarbonylgehalts eine zweite Hydrierstufe bei einem pH-Wert von 2,5 bis 6,5 bei erhöhter Temperatur anschließt. In beiden Temperaturstufen wurde der gleiche Hydrierkatalysator eingesetzt, insbesondere ein Pt/TiO₂und Ni/Al₂O₃/SiO₂-Trägerkatalysator. Es wurde festgestellt, daß sich mit einem Edelmetall auf einem oxidischen Träger in der zweiten Stufe bei einem neutralen oder schwachsauren pH-Bereich nur ein ungenügender Umsatz erzielen läßt; zudem sinkt die Katalysatoraktivität nach wenigen Betriebsstunden auf ein gegenüber dem Start niedrigeres Niveau ab.

Gemäß WO 97/01523 lassen sich cyclische Diether mit einer 1,3-Dioxogruppe in wäßriger Phase in Gegenwart eines metallischen Katalysators, wie Ru auf Aktivkohle, bei 100 bis 200 °C bei pH 1 bis 6 hydrogenolytisch spalten. Ausweislich der Beispiele ist der Umsetzungsgrad nicht quantitativ. Insbesondere gibt dieses Dokument keine Anregung, kleine Mengen Acetal oder Ketal neben großen Mengen eines Alkohols abzubauen, ohne gleichzeitig durch Hydrogenolyse auch den Alkohol zu zersetzen. Hinzu kommt, daß ein sehr hohes Verhältnis Katalysator zu Substrat erforderlich ist, um die Umsetzung zu bewirken. In ähnlicher Weise ist gemäß US-Patent 4,044,059 die hydrogenolytische Spaltung von 1,3-Dioxanderivaten unvollständig, wenn Raney Nickel als Katalysator eingesetzt wird.

Zur Reduzierung des Carbonylgehalts von 0,1 Gew.-% Laurylaldehyd enthaltendem Laurylalkohol durch katalytische Hydrierung wird laut Firmenbroschüre "Carbonyl removal from oxoalcohols" der Fa. Engelhard Corp. als Katalysator Ni auf Kisselgur empfohlen, obgleich diese Firma auch trägergebundene Edelmetallkatalysatoren im Lieferprogramm hat. Nickelkatalysatoren neigen bei pH-Werten unter 7 zu einem Metall-Leaching, was sich nachteilig auf die Aufarbeitung auswirkt.

Das Verfahren gemäß WO 97 36846 A richtet sich ausschließlich auf die Reinigung von 1,4-Butandiol.Das Reaktionsgemisch enthält 2-(4'-Hydroxybutoxy)-tetrahydrofuran. Die hydrierende Acetalspaltung erfolgt in Gegenwart von 0,5 bis 5 Gew.-% Wasser.

Im Verfahren gemäß US 2,569,671 werden Acetale enthaltende wässrige Roh-alkohole in Gegenwart von 0,005 bis 1 Gew.-% Mineralsäure durch Hydrierung gereinigt. Als Hydrierungskatalysator werden Edelmetalle genannt.

Im Verfahren des US-Patents 4,401,834 enthält das zu hydrierende Reaktionsgemisch 1 - 8 Vol.-% Wasser. Hydriert wird das in einer vorgelagerten Stufe durch thermische Spaltung eines Acetals gebildete Reaktionsgemisch, das den Aldehyd und den Alkohol enthält. Als Hydrierungskatalysatoren werden edelmetallhaltige Trägerkatalysatoren, nicht aber Aktivkohle als Träger genannt.

Im Verfahren der DE 42 20 939 A werden 2-Arylethanole aus Acetalen durch Hydrierung bei einem pH-Wert unter 6, insbesondere bei pH 1 bis 5, hergestellt.

Die hydrierende Reinigung des Acetals enthaltenden rohen primären Alkohols gemäß US 3,319,728 erfolgt zwingend zweistufig, nämlich in einer ersten Stufe bei 200 und 220°C und einer zweiten Stufe bei 150 bis 180°C. Ohne erste Stufe werden völlig unzureichende Ergebnisse bezüglich der Reduzierung des Acetalgehalts erzielt.

Aus Ind. Eng. Chem. Res. 33 (1994), 566-574 ist bekannt, organische Bestandteile, wie Zucker und Glycole, in wäßrigen Medien hydrogenolytisch in Gegenwart von Ni-Katalysatoren oder Ruthenium auf diversen oxidischen Trägern abzubauen. Bei der Verwendung derartiger Katalysatoren zur Reduzierung des Acetal- oder Ketalgehalts in einem wäßrigen Medium, das einen gegenüber dem Acetal oder Ketal vielfachen Überschuß eines Alkohols enthält, mußte im Hinblick auf dieses Dokument gleichzeitig mit einem teilweisen Abbau des Alkohols gerechnet werden.

Die WO 97/36846 betrifft ein Verfahren zur Reinigung von Butan-1,4-diol, wobei als Verunreinigung anwesendes cyclisches Acetal, 2-(4'-Hydroxybutoxy)-tetrahydrofuran, an einem Suspensions- oder Festbettkatalysator hydrierend gespalten wird.

Das im US-Patent 4,401,834 beschriebene Verfahren richtet sich auf die Gewinnung von Alkoholen aus Hydroformylierungsgemischen. An eine thermische Behandlungsstufe schließt sich eine in Abwesenheit einer Säure durchgeführte Hydrierstufe an.

Die Reinigung von Alkoholen von Aldehyden und Acetalen erfolgt gemäß US 2,569,671 durch Hydrierung in Gegenwart einer Mineralsäure und ausgewählter Hydrierkatalysatoren.

Die DE 42 20 939 A1 betrifft die Herstellung von 2-Arylethanolen aus Aryl-acetaldehydacetalen durch Hydrolyse und Hydrierung in Gegenwart von Ru-Katalysatoren.

Auch das US-Patent 3,819,728 offenbart ein Verfahren zur Reinigung eines rohen Alkohols. Das Verfahren umfasst eine zweistufige Hydrierung, nämlich zunächst bei 200 - 220 °C, dann bei 150 - 180 °C, in Gegenwart eines Trägerkatalysators.

Aufgabe der Erfindung war, ein Verfahren zur Verfügung zu stellen, womit ein geringer Acetal- oder Ketalgehalt in bei Raumtemperatur sauren oder neutralen wäßrigen Reaktionsgemischen mit hohem Gehalt an Alkoholen im wesentlichen ohne Abbau der Alkohole weiter reduziert werden kann.

Gefunden wurde ein Verfahren zur Reduzierung des Gehalts an Acetalen, ausgenommen Formalen, oder Ketalen einer α- oder β-Hydroxycarbonylverbindung in einem wässrigen Reaktionsgemisch, enthaltend pro Mol des Acetals oder Ketals mindestens 10 Mol eines ein- oder mehrwertigen Alkohols, durch katalytische Hydrierung des einen pH-Wert von 1 bis 7 aufweisenden Reaktionsgemisches mit Wasserstoff bei einer Temperatur von 80 bis 250 °C und einem H₂-Partialdruck von 0,5 bis 30 MPa, wobei als Katalysator eine Edelmetall-beladene Aktivkohle wendet wird, welches dadurch gekennzeichnet ist, dass man die katalytische Hydrierung unter Verwendung eines Katalysator-Festbetts kontinuierlich durchführt, indem man das Acetal oder Ketal und den Alkohol in gelöster Form enthaltende wässrige Reaktionsgemisch über das Festbett rieseln lässt.

Bevorzugte Ausführungsformen umfassen die Hydrierung von ein cyclisches Acetal oder Ketal mit einer 1,3-Dioxostruktur enthaltenden Reaktionsgemischen, die Verwendung von Pd oder/und Ru auf Aktivkohle als Katalysator, die Durchführung des Verfahrens unter Einsatz eines Rieselbettreaktors sowie Durchführung unter Einsatz einer Lösung mit einem pH-Wert von größer 6,5 bis 7.

Überraschenderweise ist es möglich, den Umsatz der Hydrierung des im Reaktionsgemisch enthaltenen Acetals oder Ketals bei gleicher Temperatur und gleichem pH-Wert zu steigern, ohne daß der anwesende Alkohol gleichzeitig in nenneswertem Umfang zersetzt wird, wenn anstelle eines Edelmetall-beladenen oxidischen Katalysators eine Edelmetall-beladene Aktivkohle, wie eine mit einem oder mehreren Edelmetallen aus der Reihe Ru, Rh, Pd und Pt beladene Aktivkohle, verwendet wird. Wie aus den Beispielen folgt, kommt es bei Verwendung von Ru auf TiO₂ zwar zu einer quantitativen Reaktion des Acetals, gleichzeitig aber zu einer Zersetzung des Alkohols in nicht tolerablem Umfang. Andere für die Hydrierung von Acetalen und der ihnen zugrundeliegenden Carbonylverbindung bekannte Katalysatoren, nämlich Ni auf einem SiO₂/Al₂O₃-Träger, greifen zwar den Alkohol nicht an, jedoch ist ihre Hydrieraktivität unzureichend, wenn nur eine kleine Menge Acetal neben einer großen Menge Alkohol im Reaktionsgemisch enthalten ist. Besonders bevorzugte erfindungsgemäß zu verwendende Katalysatoren sind Ru und/oder Pd auf Aktivkohle, wobei der Begriff "Aktivkohle" alle für Katalysatorzwecke geeignete Kohletypen umfaßt.

Üblicherweise erfolgt die Hydrierung bei 80 bis 180 °C. Eine gegebenenfalls auftretende Absenkung des Gehalts an Alkohol während der Hydrierung kann im allgemeinen ohne nenneswerte Minderung des Hydrierumsatzes durch Absenkung der Reaktionstemperatur vermindert oder ganz vermieden werden.

Es ist ein Vorteil der Erfindung, daß das einzusetzende Reaktionsgemisch bei Raumtemperatur einen pH-Wert von größer 6,5 bis 7 aufweisen kann, da hierdurch die Aufarbeitung vereinfacht wird.

Das der Hydrierung zuzuführende Reaktionsgemisch enthält gegenüber dem Acetal oder Ketal einen hohen molaren Überschuß an Alkohol, nämlich mindestens 10 Mol und vorzugsweise mindestens 50 Mol Alkohol pro Mol Acetal oder Ketal. Bei dem Alkohol kann es sich um einen beliebigen Alkohol handeln, da die Provenienz des Reaktionsgemischs beliebig ist; vorzugsweise handelt es sich aber um den im Acetal oder Ketal gebundenen oder/und bei der Hydrierung entstehenden Alkohol. Das Verfahren eignet sich insbesondere zur Entfernung von Acetalen, ausgenommen Formalen, oder Ketalen von α- oder β-Hydroxycarbonylverbindungen aus Reaktionsgemischen. Bei den Acetalen und Ketalen handelt es sich insbesondere um cyclische Verbindungen mit einer 1,3-Dioxostruktur, also insbesondere 1,3-Dioxan- und 1,3-Dioxolanverbindungen. Bei der hydrogenolytischen Spaltung des Rings entsteht ein 1,3-oder 1,2-Diol und der aus dem Aldehyd oder Keton gebildete Alkohol. Cyclische Acetale mit einer 1,3-Dioxostruktur, die in 2-Position nicht substituiert sind - es handelt sich hierbei um Formale - lassen sich unter üblichen Bedingungen nur teilweise hydrogenolytisch spalten.

Das Verfahren wird kontinuierlich durchgeführt und zwar unter Einsatz eines Rieselbettreaktors. Bei den hierbei zu verwendenden Festbettkatalysatoren handelt es sich bevorzugt um Pellets mit einem Durchmesser von 0,5 bis 5 mm, insbesondere 1 bis 3 mm, und einer Länge von 1 bis 10 mm. Der Edelmetallgehalt liegt im für derartige Katalysatoren üblichen Bereich, meistens 0,5 bis 5 Gew.-%.

Das erfindungsgemäße Verfahren zeichnet sich dadurch aus, daß auch ein geringer Gehalt an Acetalen oder Ketalen im gattungsgemäßen Reaktionsgemisch ohne nennenswerten Angriff des/der anwesenden gebildeten Alkohole hydrogenolytisch weiter erniedrigt werden kann. Der überraschende Effekt wird anhand der nachfolgenden Beispiele und Vergleichsbeispiele verdeutlicht.

### Beispiele

### Prinzip:

Zur kontinuierlichen Hydrierung im Rieselbett wurde eine Rieselbettanlage mit 15 ml Reaktorvolumen eingesetzt. Die Anlage bestand aus einer Flüssigkeitsvorlage, einer Vorheizstrecke, dem Festbettreaktor und einem Flüssigkeitsabscheider. Über ein Heizsystem mit Wärmeträgeröl wurde der Reaktor auf die gewünschte Reaktionstemperatur T_{R} geheizt. Der H₂-Volumenstrom betrug 13 Nl/h. Druck und Wasserstoffstrom wurden elektronisch geregelt. Die wäßrige Eduktlösung wurde vor der Vorheizstrecke dem Wasserstoffstrom mit einer Pumpe zudosiert und das Gemisch am Kopf des Reaktors aufgegeben (Rieselbettfahrweise). Nach Durchlaufen des Reaktors wurde das Reaktionsprodukt in regelmäßigen Abständen (t_{R}) aus dem Abscheider entnommen. Das Produkt wurde gaschromatographisch auf den Anteil an HED und PDO hin untersucht.

### Einsetzte Katalysatoren:

Ru/AK
   (a): Aktivkohleextrudat mit 2 % Ru; d = 2,3 mm
   (b) : " " 3 % Ru; d = 2 mm
Pd/AK: " " 2 % Pd; d = 1,5 mm
Ru/SiO₂: Granulat " 2 % Ru; d = 1-2 mm
Ru/Al₂O₃: Extrudat " 2 % Ru; d = 3 mm
Ru/TiO₂: " " 2 % Ru; d = 1 mm
Ni/SiO₂/Al₂O₃: " " 30 % Ni; d = 1,6 mm

Alle Katalysatoren, ausgenommen des handelsüblichen Ni-Katalysators, wurden nach der incipient wetness-Methode mit RuCl₃ bzw. PdCl₂ hergestellt (Preparation of Catalyst, Delmon, B. et al, Elsevier Amsterdam, 1976, Seite 13).

Die wäßrigen Einsatzlösungen der Beispiele 1 bis 10 enthielten 1,3-Propandiol (PDO) und 2-(2'-Hydroxyethyl)-1,3-dioxan (HED) und zwar 19,92 Gew.-% PDO und 0,09 Gew.-% HED in Beispiel 1 und 18,61 Gew.-% PDO und 0,09 Gew.-% HED in den Beispielen 2 bis 10. In Beispiel 11 wurde eine wäßrige Lösung von 0,1 Gew.-% 2-(2'-Hydroxyethyl)-5,5-dimethyl-1,3-dioxan (HEDD) und 20 Gew.-% Neopentylglykol (NPG) eingesetzt. In Beispiel 12 wurde eine wäßrige Lösung von 0,1 Gew.-% 2-(2-Benzoyloxyethyl)-5,5-dimethyl-1,3-dioxan (BEDD) und 20 Gew.-% Neopentylglykol eingesetzt.

Die Reaktionstemperatur T_{R}, der LHSV-Wert (liquid hourly space velocity), der pH-Wert der Eduktlösung und Analysendaten sind den Tabellen 1 und 2 zu entnehmen. Die Analysen betreffen stets Produkte, welche nach 16- bis 28-stündigem kontinuierlichen Betrieb erhalten wurden.

**Tabelle 1**

| Beispiele 1 bis 10 (LHSV: 2 h⁻¹) | | | | | |
|---|---|---|---|---|---|
| Beispiel Nr. | Katalysator | T_{R} (°C) | pH | Umsatz HED (%) | Konzentration an PDO nach der Reaktion (%) |
| 1 (comparative) | Ru/AK (a) | 130 | 4 | 92 | 20,03 |
| 2 | Ru/AK (a) | 130 | 7 | 42 | 18,58 |
| 3** | Ru/SiO₂ | 130 | 7 | 7 | 17, 03 |
| 4** | Ru/TiO₂ | 130 | 7 | 19 | 16,60 |
| 5** | Ru/Al₂O₃ | 130 | 7 | 0 | 18,22 |
| 6 | Ru/AK (b) | 130 | 7 | 30 | 18,44 |
| 7 | Pd/AK | 130 | 7 | 65 | 18,71 |
| 8** | Ni/SiO₂/ Al₂O₃ | 130 | 7 | 19 | 18, 70 |
| 9 | Ru/AK (a) | 150 | 7 | 73 | 17,03 |
| 10 | Pd/AK | 150 | 7 | 80 | 18,66 |

| | | | | | |
|---|---|---|---|---|---|
| ** nicht-erfindungsgemäße Katalysatoren | | | | | |

Aus den Beispielen folgt: Ru/AK und Pd/AK führen bei 130 °C beziehungsweise 150 °C zu einer mittleren bis guten Reduktion des Acetals HED; bei 150 °C besteht bei Ru/AK aber eine Tendenz, daß auch der Gehalt an Alkohol erniedrigt wird. Bei Ru/AK begünstigt ein schwach saurer pH-Wert die HED-Reduktion. Die oxidischen Trägerkatalysatoren führen zu einem geringen HED-Umsatz und (ausgenommen Beispiel 8) zu einem stärkeren Abbau des Alkohols.

**Tabelle 2**

| Beispiele 11 bis 13 (LHSV: 3 h⁻¹) | | | | | | | | |
|---|---|---|---|---|---|---|---|---|
| Beispiel Nr. | Reaktionsgemisch | Katalysator | T_{R} (°C) | pH | Alkohol *) | | Dioxanderivat *) | |
| | | | | | vor Reaktion | nach Reaktion | vor Reaktion | nach Reaktion |
| 11/1 | HEDD/NPG | Ru/AK (b) | 150 | 7 | 99,13 | 98,28 | 0,43 | 0,11 |
| 11/2 | " | | 130 | 7 | 99,16 | 99,08 | 0,30 | 0,09 |
| 11/3- (comparative) | " | | 150 | 5 | 99,13 | 98,61 | 0,43 | 0,08 |
| 11/4 | " | Pd/AK | 150 | 7 | 99,13 | 99,24 | 0,43 | 0,08 |
| 11/5 **) | " | Pd/TiO₂ | 150 | 7 | 99,13 | 99,13 | 0,43 | 0,37 |
| 11/6 **) | " | Ru/TiO₂ | 150 | 7 | 99,13 | 40,02 | 0,43 | 0 |
| 11/7 **) | " | Ru/SiO₂ | 150 | 7 | 99,13 | 55,52 | 0,43 | 0 |
| 11/8 **) | " | Ni/SiO₂/Al₂O₃ | 150 | 5 | 99,13 | 99,32 | 0,43 | 0,27 |
| 12 | BEDD/NPG | Ru/AK | 150 | 7 | 98,77 | 98,53 | 0,80 | 0 |
| 13 ***) | 1,3-Dioxan (0,1%) und PDO (20%) | Ru/AK | 150 | 7 | 99,31 | 97,64 | 0,55 | 0,46 |

| | | | | | | | | |
|---|---|---|---|---|---|---|---|---|
| *) Flächen-% | | | | | | | | |
| **) nicht-erfindungsgemäße Katalysatoren | | | | | | | | |
| ***) nicht-erfindungsgemäßes Reaktionsgemisch | | | | | | | | |

Erfindungsgemäß wird ein guter Abbau des Dioxanderivats bei keiner oder nur sehr geringer Zersetzung des Alkohols erzielt. Nicht-erfindungsgemäße Katalysatoren führen zu einem ungenügenden Abbau des Dioxanderivats oder zu einem guten Abbau bei gleichzeitig erheblicher Zersetzung des Alkohols. Beispiel 13 zeigt, daß sich ein geringer Gehalt eines cyclischen Formals ungenügend reduzieren läßt.

## Patentansprüche

1. Verfahren zur Reduzierung des Gehalts an Acetalen, ausgenommen Formalen, oder Ketalen einer α- oder β-Hydroxycarbonylverbindung in einem wässrigen Reaktionsgemisch, enthaltend pro Mol des Acetals oder Ketals mindestens 10 Mol eines ein- oder mehrwertigen Alkohols, durch katalytische Hydrierung des einen pH-Wert von 1 bis 7 aufweisenden Reaktionsgemisches mit Wasserstoff bei einer Temperatur von 80 bis 250 °C und einem H₂-Partialdruck von 0,5 bis 30 MPa, wobei als Katalysator eine Edelmetall-beladene Aktivkohle wendet wird,
**dadurch gekennzeichnet,**
**dass** man die katalytische Hydrierung unter Verwendung eines Katalysator-Festbetts kontinuierlich durchführt, indem man das Acetal oder Ketal und den Alkohol in gelöster Form enthaltende wässrige Reaktionsgemisch über das Festbett rieseln lässt.

2. Verfahren nach Anspruch 1,
**dadurch gekennzeichnet,**
**dass** das Reaktionsgemisch als Alkohol das durch Hydrierung der Hydroxycarbonylverbindung erhältliche 1,2- oder 1,3-Diol enthält.

3. Verfahren nach Anspruch 1 oder 2,
**dadurch gekennzeichnet,**
**dass** man als Katalysator Pd und/oder Ru auf Aktivkohle verwendet.

4. Verfahren nach einem der Ansprüche 1 bis 3,
**dadurch gekennzeichnet,**
**dass** man der katalytischen Hydrierung ein Reaktionsgemisch mit einem pH-Wert im Bereich von größer 6,5 bis 7, gemessen bei 20 °C, zuführt.

5. Verfahren nach einem der Ansprüche 1 bis 4,
**dadurch gekennzeichnet,**
**daß** man ein Reaktionsgemisch hydriert, das pro Mol Acetal oder Ketal mindestens 50 Mol des im Acetal oder Ketal gebundenen und/oder bei der Hydrierung entstehenden Alkohols enthält.

6. Verfahren nach einem der Ansprüche 1 bis 5,
**dadurch gekennzeichnet,**
**dass** man die Hydrierung bei 100 - 180 °C durchführt.

## Claims

1. A process for reducing the concentration of acetals, except for formals, or ketals of an α- or β-hydroxycarbonyl compound in an aqueous reaction mixture containing at least 10 moles per mole of the acetal or ketal of a monohydric or polyhydric alcohol by the catalytic hydrogenation of the reaction mixture of a pH of from 1 to 7 with hydrogen at a temperature of 80 to 250°C and a H₂ partial pressure of 0.5 to 30 MPa, wherein a noble metal-laden active carbon is used as catalyst,
**characterised in that**
the catalytic hydrogenation is performed continuously with the use of a catalyst fixed bed by allowing the aqueous reaction mixture containing the acetal or ketal and the alcohol in dissolved form to trickle over the fixed bed.

2. A process according to Claim 1,
**characterised in that**
the reaction mixture contains a 1,3-dioxolane derivative or 1,3-dioxan derivative as the acetal or ketal and the 1,2- or 1,3-diol obtainable by hydrogenation of the hydroxycarbonyl compound as the alcohol.

3. A process according to Claim 1 or 2,
**characterised in that**
Pd and/or Ru on active carbon is used as the catalyst.

4. A process to one of Claims 1 to 3,
**characterised in that**
a reaction mixture having a pH value in a range of greater than 6,5 to 7, measured at 20°C, is fed to the catalytic hydrogenation.

5. A process according to one of Claims 1 to 4,
**characterised in that**
a reaction mixture which contains, per mole of acetal or ketal, at least 50 moles of the alcohol bonded in the acetal of ketal and/or being produced during hydrogenation is hydrogenated.

6. A process to one of Claims 1 to 5,
**characterised in that**
the hydrogenation is performed at 100 to 180 °C.

## Revendications

1. Procédé de réduction de la teneur en acétals, à l'exception des formals, ou en cétals d'un composé α ou β - hydroxycarbonylé dans un mélange réactionnel aqueux, contenant par mole d'acétal ou de cétal, au moins 10 moles d'un alcool mono- ou pluri-fonctionnel, par hydrogénation catalytique du mélange réactionnel possédant une valeur de pH allant de 1 à 7, avec de l'hydrogène à une température allant de 80 à 250°C et à une pression partielle de H₂ de 0,5 à 30 MPa, avec comme catalyseur utilisé du charbon actif chargé avec un métal noble,
**caractérisé en ce qu'**
on effectue l'hydrogénation catalytique en continu en utilisant un lit fixe de catalyseur, et en faisant ruisseler l'acétal ou le cétal et le mélange réactionnel aqueux contenant l'alcool sous forme dissoute sur le lit fixe.

2. Procédé selon la revendication 1,
**caractérisé en ce que**
le mélange réactionnel renferme comme acétal ou cétal, un dérivé de 1,3-Dioxolane ou un dérivé de 1,3-Dioxane, et comme alcool le 1,2- ou le 1,3-Diol accessible par hydrogénation du composé hydroxycarbonylé.

3. Procédé selon la revendication 1 ou la revendication 2,
**caractérisé en ce qu'**
on utilise comme catalyseur du Pd et/ou du Ru sur charbon actif.

4. Procédé selon l'une des revendications 1 à 4,
**caractérisé en ce qu'**
on amène le mélange réactionnel possédent une valeur de pH, mesuré à 20°C, dans le domain de plus de 6,5 à 7 à l'hydrogénation catalytique.

5. Procédé selon l'une des revendications 1 à 4,
**caractérisé en ce qu'**
on hydrogénise un mélange réactionnel qui renferme par mole d'acétal ou de cétal, au moins 50 moles d'alcool lié dans l'acétal ou le cétal et/ou formé au cours de l'hydrogénation.

6. Procédé selon l'une des revendications 1 à 5,
**caractérisé en ce qu'**
on effectue l'hydrogénation à 100 à 180°C.
